# EUROPEAN PATENT APPLICATION

(11) **EP 1 571 149 A1**
(43) Date of publication of application: **07.09.2005**
(21) Application number: 04005210.2
(22) Date of filing: 05.03.2004
(51) Int. Cl.: C07D 251/68, D21H 21/30

(54) **Optical brightener solutions**

(71) Applicant: CLARIANT INTERNATIONAL LTD., 4132 Muttenz (CH)
(72) Inventor: The designation of the inventor has not yet been filed

(57) **Abstract**

The instant invention relates to concentrated aqueous solutions of hexasulphonated stilbene optical brighteners which are storage-stable in the absence of solubilizing agents like urea. By removal of salts resulting from the preparation of the optical brightener one can obtain a concentration of up to 0.350 mol/kg without losing storage stability. The reduced water content enables coating compositions which require less drying energy and which show less water and binder migration into the paper.

## Description

The instant invention relates to concentrated aqueous solutions of hexasulphonated stilbene optical brighteners which are storage-stable in the absence of solubilizing agents like urea.

### PRIOR ART

Hexasulphonated stilbene optical brightening agents (OBAs) are a well-established means of producing coated papers with a high degree of whiteness. Such optical brighteners are most conveniently marketed and used in the form of aqueous solutions.

Japanese Kokai 62-106965 discloses an optical brightener, or a salt thereof, 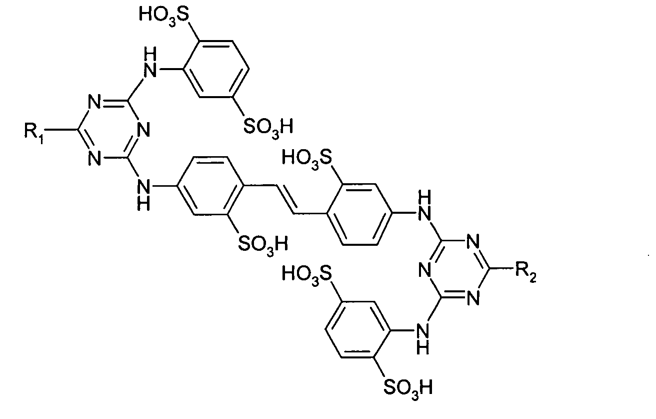 in which R₁ and R₂ each independently represents a group selected from 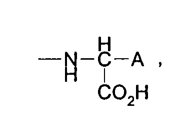 -NHCH₂CH₂CO₂H, and 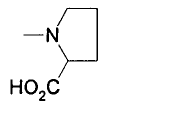 and A represents a group selected from a hydrogen atom, an alkyl group having from 1 to 4 carbon atoms, which may have a side chain, a hydroxymethyl group, a hydroxyethyl group, a methylthioethyl group, a benzyl group, a carboxymethyl group and a carboxyethyl group.

According to the Kokai, said optical brighteners have a very high solubility in water and can be commercialized in the form of a thick solution after removing the sodium chloride produced in the reaction and concentrating the water content by an appropriate method. The optical brighteners are claimed to be very effective for the whitening of paper in a surface treatment.

Each example of the Kokai describes the preparation of an optical brightener which is desalinated by ultrafiltration to give a thick solution. Supposing that in Example 1 the reaction of the starting compounds gives a 100% yield of said optical brightener then the maximal concentration in said thick solution would be 0.214 mol/kg (30-32%). Each solution is then rendered storage-stable by the addition of 10% urea.

This document therefore teaches that aqueous solutions of said optical brighteners are unstable at concentrations equal to 0.214 mol/kg (30-32%) without the addition of urea.

There is however a demand for more concentrated aqueous solutions of optical brighteners. The concentration of the aqueous solution is of particular significance when the brightener is applied in a pigmented coating composition; it is well-known that papermakers aim to minimise the water-content of the coating composition in order to minimise drying energy and to minimise water and binder migration into the base paper. (See, for example, 'The Essential Guide to Aqueous Coating of Paper and Board', ed. T. W. R. Dean, PITA, 1997.)

### DESCRIPTION OF THE INVENTION

Surprisingly and in contrast to the teaching of said Japanese document it has now been found that certain of these hexasulphonated stilbene OBAs can be prepared in the form of highly concentrated aqueous solutions that are storage-stable at concentrations of up to 0.350 mol/kg (about 54% by weight). When applied to the surface of paper in either a pigmented coating composition or in the size-press these aqueous solutions provide superior fluorescent whitening effects.

An object of the present invention therefore is a storage-stable aqueous solution comprising an optical brightener of formula (1) 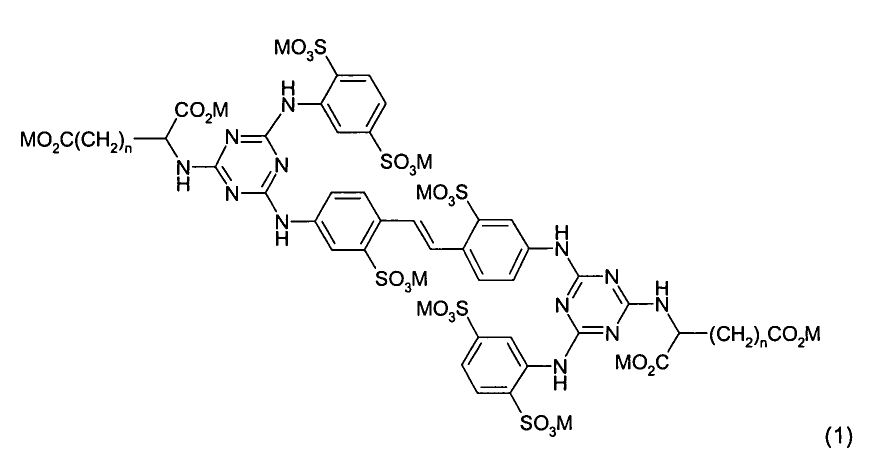 wherein
- M: is hydrogen, an alkali metal cation, ammonium, or ammonium which is mono-, di- or trisubstituted by a C₂-C₃-hydroxyalkyl radical, preferably hydrogen or a sodium cation, and
- n: is from 1 to 4, preferably is 1 or 2,
characterized in that the amount of the optical brightener is higher than 0.214 mol/kg, preferably from 0.215 to 0.350 mol/kg, more particularly from 0.250 to 0.340 mol/kg, and that no solubilizing agent is contained in the solution.

The aqueous solutions of the optical brightener(s) may optionally contain one or more carriers, antifreezes, preservatives, complexing agents etc., as well as organic byproducts formed during the preparation of the optical brightener.

Carriers are known to give improved whitening characteristics and may be, e.g., polyethylene glycols, polyvinyl alcohols or carboxymethylcelluloses.

Antifreezes may be, e.g., urea, diethylene glycol or triethylene glycol.

The compounds of formula (1) are prepared by stepwise reaction of a cyanuric halide with
a) a diamine of formula (A) 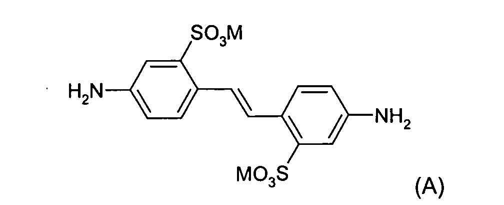
b) an amine of formula (B) 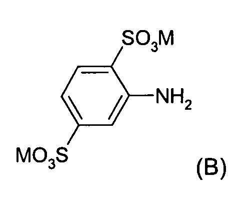 and
c) an amine of formula (C) 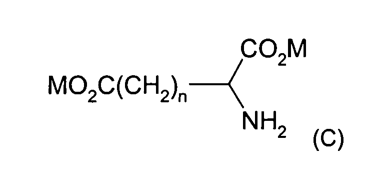
As a cyanuric halide there may be employed the bromide or, preferably, the chloride.

Each reaction may be carried out in an aqueous medium, the cyanuric halide being suspended in water, or in an aqueous/organic medium, the cyanuric halide being dissolved in a solvent such as acetone. Each amine may be introduced without dilution, or in the form of an aqueous solution or suspension. The amines can be reacted in any order, although it is preferred to react the aromatic amines first. Each amine may be reacted stoichiometrically, or in excess. Typically, the aromatic amines are reacted stoichimetrically, or in slight excess; the aliphatic amines are generally employed in an excess of 5-30% over stoichiometry.

For substitution of the first halogen of the cyanuric halide, it is preferred to operate at a temperature in the range of 0 to 20°C, and under acidic to neutral pH conditions, preferably in the pH range of 2 to 7. For substitution of the second halogen of the cyanuric halide, it is preferred to operate at a temperature in the range of 20 to 60°C, and under weakly acidic to weakly alkaline conditions, preferably at a pH in the range of 4 to 8. For substitution of the third halogen of the cyanuric halide, it is preferred to operate at a temperature in the range of 60 to 102°C, and under weakly acidic to alkaline conditions, preferably at a pH in the range of 7 to 10. The pH may be controlled by addition of suitable acids or bases as necessary, preferred acids being e.g., hydrochloric acid, sulphuric acid, formic acid or acetic acid, preferred bases being e.g., alkali metal (e.g., lithium, sodium or potassium) hydroxides, carbonates or bicarbonates, or aliphatic tertiary amines e.g. triethanolamine or triisopropanolamine.

A further object of the instant invention is the process for the preparation of said aqueous solutions wherein the compounds of formula (1) are prepared as described above and wherein the alkali metal or amine salt that is generated as a by-product of each reaction between an amine and a cyanuric halide is removed from the reaction solution.

In order to prepare a stable aqueous solution with a concentration of higher than 0.214 mol/kg (32-33% by weight), preferably from 0.214 to 0.350 mol/kg, more particularly from 0.250 to 0.340 mol/kg, of a compound of formula (1) without the addition of solubilizing aids such as ethylene glycol, urea or a mono-, di- or tri-(2-hydroxyethyl)- or (2-hydroxypropyl)-amine, it is necessary to remove at least 50 %, preferably at least 80 % by weight, of the alkali metal or amine salt that is generated as a by-product of each reaction between an amine and a cyanuric halide. This is preferably done by ultrafiltration or membrane filtration of the solution formed as described above. Alternatively, the compound of formula (1) can be isolated by precipitation (e.g. by the addition of acid) then redissolved.

A further object of the instant invention is the use of the instant storage-stable aqueous solutions for brightening of paper or other cellulosic substrates.

The concentrated solutions of compounds of formula (1) are particularly suitable for the brightening of paper after sheet formation. This may be effected by adding the optical brightener solution to a pigmented coating composition, or to a sizing solution or suspension.

In a preferred aspect to the instant invention, the concentrated solutions of compounds of formula (1) are applied to the surface of paper in a pigmented coating composition.

The coating compositions are essentially aqueous compositions that contain at least one binder and a white pigment, in particular an opacifying white pigment, and may additionally contain further additives such as dispersing agents and defoamers.

Although it is possible to produce coating compositions that are free from white pigments, the best white substrates for printing are made using opaque coating compositions that contain 10-70% white pigment by weight. Such white pigments are generally inorganic pigments, e.g., aluminium silicates (kaolin, otherwise known as china clay), calcium carbonate (chalk), titanium dioxide, aluminium hydroxide, barium carbonate, barium sulphate, or calcium sulphate (gypsum).

The binders may be any of those commonly used in the paper industry for the production of coating compositions and may consist of a single binder or of a mixture of primary and secondary binders. The sole or primary binder is preferably a synthetic latex, typically a styrene-butadiene, vinyl acetate, styrene acrylic, vinyl acrylic or ethylene vinyl acetate polymer. The secondary binder may be, e.g., starch, carboxymethylcellulose, casein, soy polymers, or polyvinyl alcohol.

The sole or primary binder is used in an amount typically in the range 5-25% by weight of white pigment. The secondary binder is used in an amount typically in the range 0.1-2% by weight of white pigment; starch however is typically used in the range 5-10% by weight of white pigment.

The optical brightener of formula (1) is used in an amount typically in the range 0.01-1% by weight of white pigment, preferably in the range 0.05-0.5% by weight of white pigment.

The following examples shall explain the instant invention in more detail. "Parts" means, if not defined differently, "parts by weight".

### PREPARATIVE EXAMPLE 1

A solution of 21.3 parts aniline-2,5-disulphonic acid and 6.7 parts sodium hydroxide in 30 parts water is added to a stirred suspension of 15.5 parts cyanuric chloride in 50 parts ice water. The pH is kept at 6 by the dropwise addition of 30% sodium hydroxide. The mixture is stirred below 10°C until primary aromatic amine groups can no longer be detected by the diazo reaction. A solution of 14.8 parts 4,4'-diaminostilbene-2,2'-disulphonic acid and 3.2 parts sodium hydroxide in 20 parts water is then added, the pH is adjusted to between 6.5 and 7.5 by the addition of 30% sodium hydroxide and the mixture is stirred at 30°C until a negative diazo reaction is obtained. A solution of 12.2 parts L-aspartic acid in 22.9 parts 16% sodium hydoxide is added, and the mixture is heated at reflux for 6 hours, the pH being kept at 7.5 to 8.5 by the addition of 30% sodium hydroxide. The aqueous solution so-formed of (2) in the form of its sodium salt, which is approximately 0.167 mol/kg in concentration, is desalinated by membrane filtration and concentrated to 0.330 mol/kg (50% by weight). The resulting solution, 2.0% in sodium chloride, has a viscosity of 0.23-0.25 Pa.s at 20°C and shows no signs of precipitation after 2 weeks at 5°C.

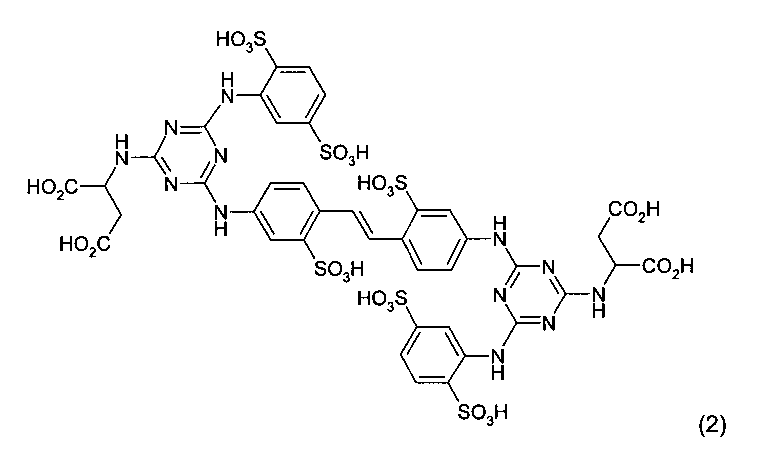

### PREPARATIVE EXAMPLE 2

An aqueous solution of (3) in the form of its sodium salt at a concentration of 0.330 mol/kg (51 % by weight) is prepared as described in Example 1 but using 13.5 parts L-glutamic acid in place of 12.2 parts L-aspartic acid. The resulting solution, 2.0 % in sodium chloride, has a viscosity of 0.23-0.25 Pa.s at 20°C and shows no signs of precipitation after 2 weeks at 5°C.

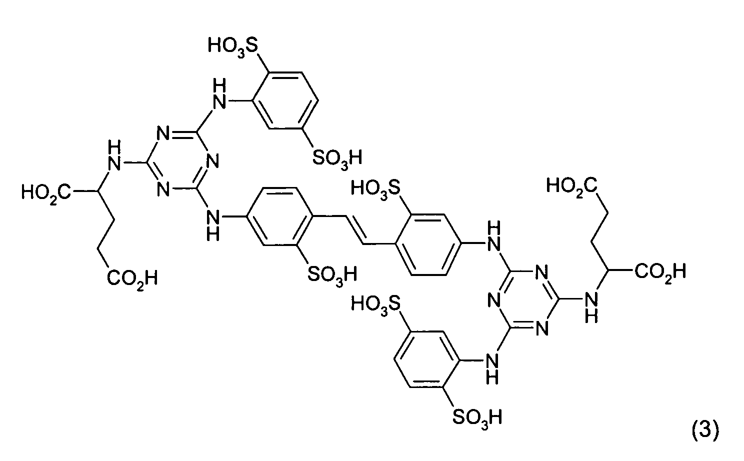

### APPLICATION EXAMPLE 1.

A coating composition is prepared containing 500 parts chalk (commercially available under the trade name Hydrocarb 90 from OMYA), 500 parts clay (commercially available under the trade name Kaolin SPS from IMERYS), 470 parts water, 6 parts dispersing agent (a sodium salt of a polyacrylic acid commercially available under the trade name Polysalz S from BASF), 200 parts latex (an acrylic ester copolymer commercially available under the trade name Acronal S320D from BASF) and 30 parts of a 10% solution of carboxymethyl cellulose (commercially available under the trade name Finnfix 5.0 from Noviant) in water. The solids content is adjusted to 65% by the addition of water, and the pH is adjusted to 8-9 with sodium hydroxide.

The solution of brightener (2) in the form of its sodium salt, made as described in Preparative Example 1, is added at a range of concentrations from 0.1 to 0.6% to the stirred coating composition. The brightened coating composition is then applied to a commercial 75 gsm neutral-sized white paper base sheet using an automatic wire-wound bar applicator with a standard speed setting and a standard load on the bar. The coated paper is then dried for 5 minutes in a hot air flow. The dried paper is allowed to condition, then measured for CIE Whiteness on a calibrated Elrepho spectrophotometer.

**TABLE 1**

| **OBA Concentration (%)** | **CIE Whiteness using Brightener of Example 1** |
|---|---|
| 0 | 92.0 |
| 0.1 | 97.9 |
| 0.2 | 102.6 |
| 0.3 | 106.2 |
| 0.4 | 108.4 |
| 0.5 | 110.3 |
| 0.6 | 112.2 |

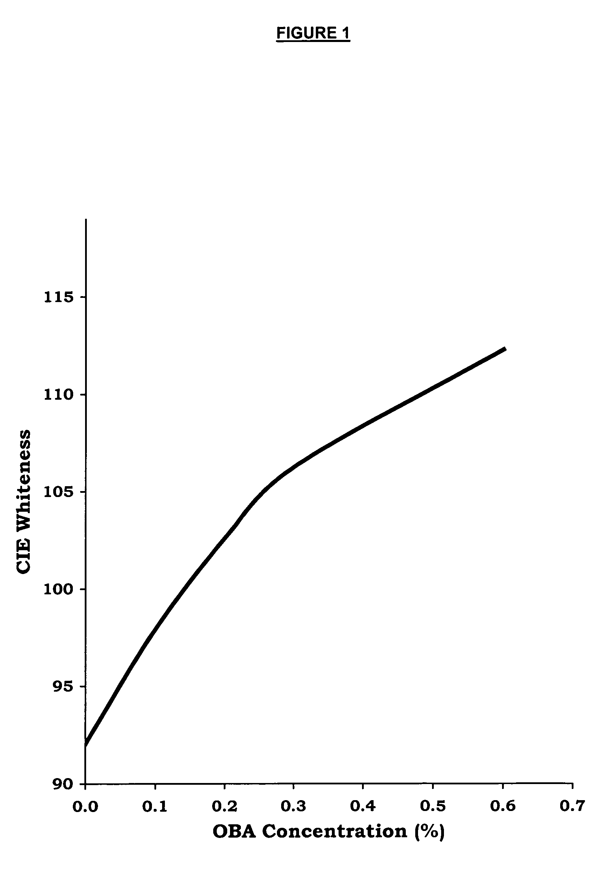

The results are also shown in graphical form in Figure 1.

## Claims

1. Storage-stable aqueous solution comprising an optical brightener of formula (1) 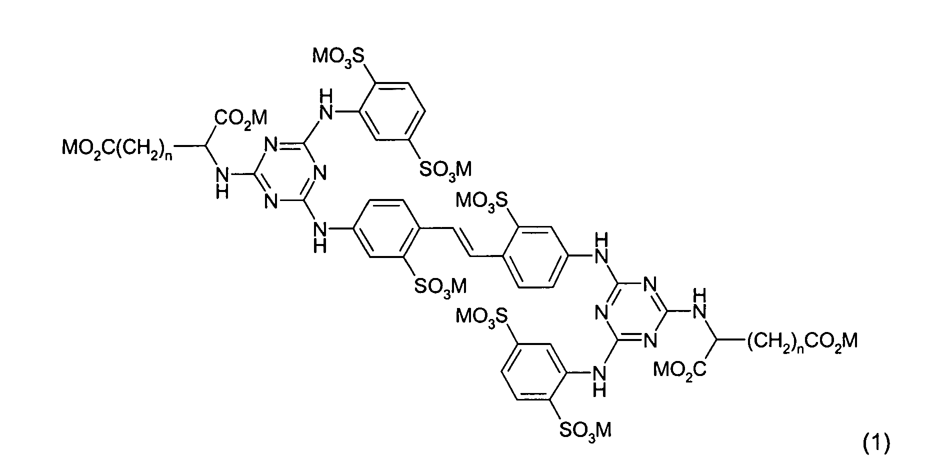 wherein
M is hydrogen, an alkali metal cation, ammonium, or ammonium which is mono-, di- or trisubstituted by a C₂-C₃-hydroxyalkyl radical, and
n is from 1 to 4,
**characterized in that** the amount of the optical brightener is higher than 0.214 mol/kg and that no solubilizing agent is contained in the solution.

2. Storage-stable aqueous solution according to claim 1 wherein
M is hydrogen or a sodium cation, and
n is 1 or 2.

3. Storage-stable aqueous solution according to claim 1 or 2 wherein the concentration of the optical brightener is from 0.215 mol/kg to 0.350 mol/kg.

4. Storage-stable aqueous solution according to claim 3 wherein the concentration of the optical brightener is from 0.250 mol/kg to 0.340 mol/kg.

5. Storage-stable aqueous solution according to any of claims 1 to 4 wherein additionally inorganic salts, carriers, antifreezes, preservatives or complexing agents are contained.

6. Process for the preparation of an aqueous solution according to any of claims 1 to 5 wherein the compounds of formula (1) are prepared by stepwise reaction of a cyanuric halide with
a) a diamine of formula (A) 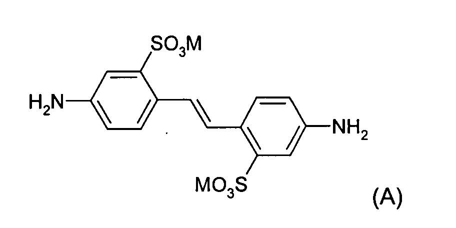
b) an amine of formula (B) 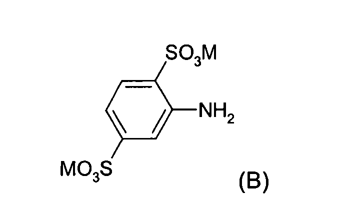 and
c) an amine of formula (C) 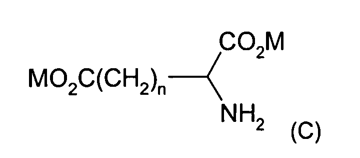 and wherein at least 50 %, preferably at least 80 % by weight, of the alkali metal or amine salt that is generated as a by-product of each reaction between an amine and a cyanuric halide is removed from the reaction solution.

7. Process according to claim 6 wherein the removal of the alkali metal or amine salt is done by ultrafiltration or membrane filtration of the reaction solution or by isolating the optical brightener and then dissolving it again.

8. Process according to claim 7 wherein the removal is done by membrane filtration.

9. Use of a storage-stable aqueous solution according to any of claims 1 to 5 for brightening of paper or other cellulosic substrates wherein the optical brightener is used in a concentration of 0.05 to 0.5 % by weight of the white pigment..

10. Use according to claim 9 for brightening of paper in a pigmented coating composition.
